# EUROPEAN PATENT SPECIFICATION

(11) **EP 1 473 029 B1**
(45) Date of publication and mention of the grant of the patent: **23.03.2005**
(21) Application number: 03009659.8
(22) Date of filing: 30.04.2003
(51) Int. Cl.: A61K 9/20, A61K 38/11

(54) **Solid dosage form comprising desmopressin**
Feste Dosierungsform mit Desmopressin
Forme posologique solide de la desmopressine

(43) Date of publication of application: 03.11.2004
(73) Proprietor: Ferring B.V., 2132 JX Hoofddorp (NL)
(72) Inventor: Lomryd, Hakan, 215 62 Malmö (SE); Nicklasson, Helena, 217 59 Malmö (SE); Olsson, Lars-Erik, 215 67 Malmö (SE)
(74) Representative: HOFFMANN EITLE

(56) References cited:
- WO-A-00/59423
- WO-A-97/15297
- US-A- 5 047 398

## Description

### Field of the Invention

The present invention relates to a novel pharmaceutical composition as a solid dosage form comprising desmopressin as a therapeutically active ingredient, and to a method for manufacturing thereof.

### Technical Background

Desmopressin, also known as dDAVP, is the therapeutically active ingredient (as its acetate salt) in the pharmaceutical product Minirin®, which is marketed *inter alia* as a nasal spray and a tablet formulation. Desmopressin is primarily used in the treatment of primary nocturnal enuresis, *i.e.* bedwetting, in children, but it is approved also for the treatment of nocturia and diabetes insipidus. The first market introduction of the tablet formulation was in Sweden in 1987.

In short, a solid dosage form such as a tablet formulation is typically manufactured by compression of a suitable granulate to the desired solid dosage form, where the granulate is composed of the required constituents as a mixture of solid particles. Typical such particles are the therapeutically active ingredient, various excipients (fillers), disintegrating agents, lubricants and binders, optionally together *e.g.* with flavoring agent, preservative and/or colorant. The commercially available Minirin® tablet is prepared according to this general protocol, and the tablet was first disclosed as set forth in the patent US 5 047 398. For a comprehensive overview of pharmaceutical tablet manufacturing, see *"Tableting"* (by N.A. Armstrong) in *"Pharmaceutics - The science of dosage form design",* pp 647-668; Ed. M.E. Aulton, Churchill Livingstone, Edinburgh, London, Melbourne and New York, 1988.

The Minirin® tablet that is currently marketed, and thus produced in industrial scale, consists of the therapeutically active ingredient desmopressin together with potato starch and lactose as excipients, and a suitable amount of binder and lubricant, respectively.

In any tablet compression of a granulate composed of a mixture of solid particles there is a general need to perform the compressing operation at the highest possible speed while at the same time minimising machine wear and obtaining tablets of a quality that meets the regulatory demands of all relevant territories.

### Disclosure of the Invention

It has now been discovered that a certain dimension of the excipient particles unexpectedly provides a substantial improvement on the speed of the manufacturing process, while both machine wear and tablet quality remain substantially unaltered compared to the industrial manufacturing process hitherto used. In essence, the dimension in question seems to affect the flowability of particles and granulate in such a manner that it provides an improved overall capacity, and hence speed, in the manufacturing process for the desmopressin tablet formulation.

More specifically, the present invention relates to a pharmaceutical composition as a solid dosage form comprising desmopressin, or a pharmaceutically acceptable salt thereof, as a therapeutically active ingredient together with a pharmaceutically acceptable excipient, diluent or carrier, or mixture thereof, wherein at least one of said excipient, diluent and carrier is a disaccharide wherein the said disaccharide has an average particle size in the range of from 70 to 500 µm.

In many cases the terms excipient, diluent and carrier can be used interchangeably, and they may even refer to one and the same substance, or to a mixture of similar such substances. The proper use and understanding of these terms is self-explanatory and lies well within the ability of a person skilled in the art of pharmaceutical formulation.

The pharmaceutical composition according to the present invention may optionally comprise at least one additive selected from a disintegrating agent, lubricant, binder, flavoring agent, preservative, colorant and a mixture thereof. Where considered suitable also other additives may be included. Representative examples of disintegrating agents, lubricants (*e.g.* magnesium stearate), binders (*e.g*. Kollidon® 25, BASF), flavoring agents, preservatives and colorants, and suitable mixtures thereof, as well as any other conventional additive that may be considered by a person skilled in the art practising the present invention, can be found in *"Handbook of Pharmaceutical Excipients";* Ed. A.H. Kibbe, 3^{rd} Ed., American Pharmaceutical Association, USA and Pharmaceutical Press UK, 2000. As an example, also applicable in the practising of the present invention, it can be mentioned that typical amounts of lubricants and binders are in the order of less than 6 percent by weight of the pharmaceutical composition.

In the marketed tablet resulting from the hitherto used manufacturing process, the lactose particles (Pharmatose 150M provided by DMV, the Netherlands) have an average size of about 50 µm, as determined by an air jet sieve (provided by Alpine GmbH, DE). That particle size does not provide a granulate that allows a compressing speed exceeding about 170 000 tablets per hour (h). In contrast thereto, the process according to the present invention allows a compressing speed of up to about 250 000 tablets/h with the desired tablet quality and retained low level of wear on the tabletting machinery.

In preferred embodiment, said average particle size is in the range of from 75 to 350 µm. In yet another preferred embodiment, said average particle size is in the range of from 100 to 200 µm. In a further preferred embodiment, said average particle size is in the range of from 120 to 180 µm. In the most preferred embodiment of the present invention, said average particle size is 140 µm (as measured by an air jet sieve). The lactose particles sold as Pharmatose DCL 15, marketed by DMV in the Netherlands, are of this most preferred average particle size. Other particular embodiments may involve use of *e.g.* Pharmatose DCL 11, Pharmatose DCL 21 and Pharmatose DCL 40, all provided by the aforementioned DMV, which have an average particle size of 110, 150 and 165 µm, respectively.

According to the commercial provider the particle size distribution of Pharmatose DCL 15 is that essentially all particles have a size below 500 µm, whereas approximately 72% of the particles have a size of from 75 to 350 µm.

In an air jet sieve measurement of particle size, air is drawn upwards, through a sieve, from a rotating slit so that material on the sieve is fluidised. At the same time a negative pressure is applied to the bottom of the sieve which removes fine particles to a collecting device. Size analyses and determination of average particle size are performed by removal of particles from the fine end of the size distribution by using single sieves consecutively. See also *"Particle Size Measurement"*, 5^{th} Ed., p 178, vol. 1; T. Allen, Chapman & Hall, London, UK, 1997, for more details on this. For a person skilled in the art, the size measurement as such is thus of conventional character.

Accordingly, it is preferred that said disaccharide is lactose, more preferably lactose-α-monohydrate.

The total combined amount of said excipient, diluent and carrier is usually from 5 to 99, preferably from 50 to 99, percent by weight of the pharmaceutical composition, the balance up to 100% being the therapeutically active ingredient optionally together with the aforementioned additives, where the latter is preferably lubricant and binder.

The pharmaceutical composition as a solid dosage form according to the present invention is typically a perorally available tablet. As an alternative nonlimiting embodiment, the said tablet may be adapted for oral, including buccal and/or sublingual, administration.

The composition typically comprises desmopressin acetate in an amount of from 20 to 600 µg per unit of solid dosage form. As an example, a typical tablet containing 100 µg of desmopressin acetate is white, convex and oval (6.7 x 9.5 mm) with a thickness of 3-4 mm and a weight of 200 mg. As another example, a tablet containing 200 µg of desmopressin acetate is white, round (8 mm diameter) and convex with a thickness of 3-4 mm and a weight of 200 mg.

Accordingly, a further aspect of the present invention relates to a method for the manufacturing of a pharmaceutical composition as a solid dosage form comprising desmopressin, or a pharmaceutically acceptable salt thereof, as a therapeutically active ingredient, wherein said method comprises the steps of:
i) mixing desmopressin and an excipient, diluent or carrier, or mixture thereof, optionally in the presence of a wetting agent, wherein at least one of said excipient, diluent and carrier is a disacchariade, wherein said disaccharide has an average particle size in the range of from 70 to 500 µm;
ii) subjecting the resulting mixture to formation of a granulate, optionally in the presence of a wetting agent, suitable for compression into said solid dosage form;
iii) optionally performing said mixing and/or formation of a granulate in the presence of at least one additive selected from a disintegrating agent, lubricant, binder, flavoring agent, preservative, colorant and a mixture thereof;
iv) optionally drying said granulate;
v) compressing said granulate into said solid dosage form.

The method according to the present invention can as such, once the specific components are identified and included, be practised by using conventional equipment for the manufacturing of pharmaceutical formulations. A granulate suitable for compression into tablets typically has an average granulate size of at least about 100 µm. Discrete granules with a size above 2 mm are usually not transferred to the subsequent compressing step.

As examples mention can be made of the following equipment for granulation: directly heated fluidised solid beds *e.g.* provided by GEA/Collette NV, BE (UltimaPro™ series) , Hüttlin GmbH, DE (HDG series), Diosna Dierks & Soehne GmbH, DE (VAC series), Fluid Air Inc., US (Magnaflo® series) and Vector Corp., US (GMX series); indirect conduction moving solids bed, including paddle systems, rotary systems and agitation systems, which are *e.g.* provided by Jaygo Inc., US (JRB and Novamix series), Paul O. Abbé Inc., US (Rota-Cone, Rota-U, Rota Blade, Cylindrical Ribbon/Paddle, Plow and Sigmablade series), Forberg A/S, NO (Forberg II series), Gemco Inc., US (D/3 Double Cone, V-Shape and Slant-Cone series), LittlefordDay Inc., US (Double Arm, Day Nauta and Daymax series), Patterson-Kelly, Harsco Corp., US (P-K Solids Processor@ series), Diosna as above (CCS and VAC series), Romaco Zanchetta SpA, IT (Roto E, Roto D and Roto P series) and L.B. Bohle Maschinen und Verfahren GmbH, DE (Granumator GMA and Vagumator VMA series); The aforementioned equipment in general also provides drying of the prepared granules.

In preferred embodiment, said average particle size is in the range of from 75 to 350 µm. In yet another preferred embodiment, said average particle size is in the range of from 100 to 200 µm. In a further preferred embodiment, said average particle size is in the range of from 120 to 180 µm. In the most preferred embodiment of the present invention, said average particle size is 140 µm. The lactose particles sold as Pharmatose DCL 15, marketed by DMV in the Netherlands, are of this most preferred average particle size. Other possible embodiments of the present method may involve the aforementioned variants of Pharmatose DCL (*vide supra*).

It is accordingly preferred that said disaccharide is lactose, more preferably

In the method according to the present invention, the manufactured solid dosage form is typically a perorally available tablet. Where desired, it may also be in a form and/or composition adapted for oromucosal administration. Preferred examples of the latter are buccal and/or sublingual administration. Examples of tablet compressing equipment suitable for the practising of the present invention are rotary presses provided by Elizabeth-Hata International, US (HT series), Courtoy NV, BE (R090F, R100M, R190FT, R290FT, R292F and R233 series), Vector Corp., US (2000, 200 and Magna series), Fette GmbH, DE (Hightech, Medium, Special and WIP series), Manesty, UK (Xpress, Diamond and Value series) and Kilian & Co. GmbH, DE (S, T, E, RX and KTS series).

In a preferred embodiment of the present inventive method said steps of mixing and formation of granulate are performed in a single integrated machinery that is adapted for such a "one-pot", *i.e.* combined, process. An example of such integrated machinery, alternatively denoted one-pot (single pot) equipment, is the FT series, provided by Forberg A/S, Norway.

It is preferred that where used, said wetting agent is selected from water and a mixture of water and an alcohol, preferably ethanol. A water/ethanol 1:3 mixture is typically used, albeit many other combinations are also possible.

As indicated above, it is preferred that said resulting mixture is subjected to formation of a granulate with an average granulate size of a least 100 µm, preferably in the range of from 100 µm to 2 mm.

The method is preferably performed in such a manner that the total combined amount of said excipient, diluent and carrier is from 5 to 99, preferably from 50 to 99, percent by weight of the pharmaceutical composition.

In the most preferred embodiment, desmopressin acetate is used and mixed with said excipient, diluent and/or carrier in an amount that eventually provides from 20 to 600 µg of desmopressin acetate per unit of solid dosage form (see above and the experimental part for examples of a tablet).

In order to substantiate and illustrate the present invention in more detail, the following example is provided.

### Example

### Example 1: Preparation of solid dosage form of dDAVP

Desmopressin acetate (100 or 200 g; provided by PolyPeptide Laboratories AB, SE), polyvinyl pyrrolidone (PVP) as binder (1,84 kg; Kollidon® 25 provided by BASF GmbH, DE) and granulation liquid (water/ethanol 1:3 mixture) are combined in a vessel and mixed at room temperature until a clear solution is achieved. The potato starch (77 kg, average particle size about 40-50 µm according to laser diffraction measurements; AmylSolVät provided by Lyckeby Stärkelse AB, SE), is weighed and sieved through a 2 mm sieve. Lactose (120 kg, DCL 15 provided by DMV NV, NL; see above for the details of this product) is weighed and loaded together with the starch into a single pot mixer (FT-350; provided by Forberg A/S, NO) and mixed therein. The granulation liquid solution is then sprayed onto the powder mixture, after which the moist granulate is dried with warm air (150°C), all with continued mixing. The dried granulate is then sieved (2 mm) and transferred to a double cone mixer. Magnesium stearate (max 1.0 kg; provided by Peter Greven NV, NL) is then weighed in, sieved (1 mm) and transferred to the double cone mixer for final mixing. Tablets are then compressed from the resulting mixture by using a conventional rotary tablet compression machine (Kilian S-250), whereby a compressing speed of about 250 000 tablets/h is attainable with adequate tablet quality and low machine wear. A tablet of adequate quality has a smooth surface without scratches or chipped edges, and it shows no tendencies to lamination (so-called capping).

The process is typically adapted to provide a tablet containing 100 or 200 µg of desmopressin acetate with the aforementioned appearance, dimension and weight.

## Claims

1. A pharmaceutical composition as a solid dosage form comprising desmopressin, or a pharmaceutically acceptable salt thereof, as a therapeutically active ingredient together with a pharmaceutically acceptable excipient, diluent or carrier, or mixture thereof, wherein at least one of said excipient, diluent and carrier is a disaccharide, wherein said disaccharide has an average particle size in the range of from 70 to 500 µm.

2. The pharmaceutical composition according to claim 1, wherein said average particle size is in the range of from 75 to 350 µm.

3. The pharmaceutical composition according to claim 2, wherein said average particle size is in the range of from 100 to 200 µm.

4. The pharmaceutical composition according to claim 3, wherein said average particle size is in the range of from 120 to 180 µm.

5. The pharmaceutical composition according to any of claims 1 to 4, wherein said disaccharide is lactose, preferably lactose-α-monohydrate.

6. The pharmaceutical composition according to any of claims 1 to 5, wherein the total combined amount of said excipient, diluent and carrier is from 5 to 99, preferably from 50 to 99, percent by weight of the pharmaceutical composition.

7. The pharmaceutical composition according to any one of claims 1 to 6, wherein said solid dosage form is a perorally available tablet that is optionally adapted for oromucosal, preferably buccal and/or sublingual, administration.

8. The pharmaceutical composition according to any one of claims 1 to 7, which comprises desmopressin acetate in an amount of from 20 to 600 µg per unit of solid dosage form.

9. A method for the manufacture of a pharmaceutical composition as a solid dosage form comprising desmopressin, or a pharmaceutically acceptable salt thereof, as a therapeutically active ingredient, wherein said method comprises the steps of:
i) mixing desmopressin and an excipient, diluent or carrier, or mixture thereof, optionally in the presence of a wetting agent, wherein at least one of said excipient, diluent and carrier is a disaccharide, wherein said disaccharide has an average particle size in the range of from 70 to 500 µm;
ii) subjecting the resulting mixture to formation of a granulate, optionally in the presence of a wetting agent, suitable for compression into said solid dosage form;
iii) optionally performing said mixing and/or formation of a granulate in the presence of at least one additive selected from a disintegrating agent, lubricant, binder, flavoring agent, preservative, colorant and a mixture thereof;
iv) optionally drying said granulate;
v) compressing said granulate into said solid dosage form.

10. The method according to claim 9, wherein said average particle size is in the range of from 75 to 350 µm.

11. The method according to claim 10, wherein said average particle size is in the range of from 100 to 200 µm.

12. The method according to claim 11, wherein said average particle size is in the range of from 120 to 180 µm.

13. The method according to any one of claims 9 to 12, wherein said disaccharide is lactose, preferably lactose-α-monohydrate.

14. The method according to any one of claims 9 to 13, wherein said solid dosage form is a perorally available tablet that is optionally adapted for oromucosal, preferably buccal and/or sublingual, administration.

15. The method according to any one of claims 9 to 14, wherein said steps of mixing and formation of a granulate are performed in a single integrated machinery that is adapted for such a combined process.

16. The method according to any one of claims 9 to 15, wherein said wetting agent is selected from water and a mixture of water and an alcohol, preferably ethanol.

17. The method according to any one of claims 9 to 16, wherein said resulting mixture is subjected to formation of a granulate with an average granulate size of a least 100 µm, preferably in the range of from 100 µm to 2 mm.

18. The method according to any one of claims 9 to 17, wherein the total combined amount of said excipient, diluent and carrier is from 5 to 99, preferably from 50 to 99, percent by weight of the pharmaceutical composition

19. The method according to any one of claims 9 to 18, wherein desmopressin acetate is used and mixed with the excipient, diluent or carrier in an amount that provides from 20 to 600 µg of desmopressin acetate per unit of solid dosage form.

## Patentansprüche

1. Pharmazeutische Zusammensetzung als feste Arzneiform, die Desmopressin oder ein pharmazeutisch akzeptables Salz davon als therapeutisch wirksamen Bestandteil zusammen mit einem pharmazeutisch akzeptablen Exzipienten, Verdünnungsmittel oder Träger oder einer Mischung daraus umfasst, worin wenigstens ein Vertreter aus dem Exzipienten, Verdünnungsmittel und Träger ein Disaccharid ist, worin das Disaccharid eine durchschnittliche Teilchengröße im Bereich von 70 bis 500 µm hat.

2. Pharmazeutische Zusammensetzung gemäß Anspruch 1, worin die durchschnittliche Teilchengröße im Bereich von 75 bis 350 µm ist.

3. Pharmazeutische Zusammensetzung gemäß Anspruch 2, worin die durchschnittliche Teilchengröße im Bereich von 100 bis 200 µm ist.

4. Pharmazeutische Zusammensetzung gemäß Anspruch 3, worin die durchschnittliche Teilchengröße im Bereich von 120 bis 180 µm ist.

5. Pharmazeutische Zusammensetzung gemäß einem der Ansprüche 1 bis 4, worin das Disaccharid Lactose ist, bevorzugt Lactose-α-Monohydrat.

6. Pharmazeutische Zusammensetzung gemäß einem der Ansprüche 1 bis 5, worin die kombinierte Gesamtmenge aus dem Exzipienten, Verdünnungsmittel und Träger 5 bis 99, bevorzugt 50 bis 99 Gew.-% der pharmazeutischen Zusammensetzung beträgt.

7. Pharmazeutische Zusammensetzung gemäß einem der Ansprüche 1 bis 6, worin die feste Arzneiform eine peroral verfügbare Tablette ist, die gegebenenfalls zur oromukosalen, bevorzugt bukkalen und/oder sublingualen, Verabreichung angepasst ist.

8. Pharmazeutische Zusammensetzung gemäß einem der Ansprüche 1 bis 7, die Desmopressinacetat in einer Menge von 20 bis 600 µg pro Einheit der festen Arzneiform umfasst.

9. Verfahren zur Herstellung einer pharmazeutischen Zusammensetzung als feste Arzneiform, die Desmopressin oder ein pharmazeutisch akzeptables Salz davon als therapeutisch wirksamen Bestandteil umfasst, worin das Verfahren die folgenden Schritte umfasst:
(i) Vermischen von Desmopressin und einem Exzipienten, Verdünnungsmittel oder Träger oder einer Mischung daraus, gegebenenfalls in Gegenwart eines Benetzungsmittels, worin wenigstens ein Vertreter aus dem Exzipienten, Verdünnungsmittel und Träger ein Disaccharid ist, worin das Disaccharid eine durchschnittliche Teilchengröße im Bereich von 70 bis 500 µm hat;
(ii) Unterwerfen der resultierenden Mischung der Bildung eines Granulats, gegebenenfalls in Gegenwart eines Benetzungsmittels, geeignet zum Verpressen zur festen Arzneiform;
(iii) Gegebenenfalls Durchführen des Vermischens und/oder der Bildung eines Granulats in Gegenwart wenigstens eines Additivs, das aus einem Sprengmittel, Schmiermittel, Bindemittel, Geschmacksmittel, Konservierungsmittel, Farbstoff und einer Mischung daraus ausgewählt ist;
(iv) Gegebenenfalls Trocknen des Granulats;
(v) Verpressen des Granulats zur festen Arzneiform.

10. Verfahren gemäß Anspruch 9, worin die durchschnittliche Teilchengröße im Bereich von 75 bis 350 µm ist.

11. Verfahren gemäß Anspruch 10, worin die durchschnittliche Teilchengröße im Bereich von 100 bis 200 µm ist.

12. Verfahren gemäß Anspruch 11, worin die durchschnittliche Teilchengröße im Bereich von 120 bis 180 µm ist.

13. Verfahren gemäß einem der Ansprüche 9 bis 12, worin das Disaccharid Lactose ist, bevorzugt Lactose-α-Monohydrat.

14. Verfahren gemäß einem der Ansprüche 9 bis 13, worin die feste Arzneiform eine peroral verfügbare Tablette ist, die gegebenenfalls zur oromukosalen, bevorzugt bukkalen und/oder sublingualen, Verabreichung angepasst ist.

15. Verfahren gemäß einem der Ansprüche 9 bis 14, worin die Schritte aus Vermischen und Bilden eines Granulats in einer einzigen integrierten Ausrüstung durchgeführt werden, die für einen solchen kombinierten Prozess angepasst ist.

16. Verfahren gemäß einem der Ansprüche 9.bis 15, worin das Benetzungsmittel aus Wasser und einer Mischung aus Wasser und einem Alkohol, bevorzugt Ethanol, ausgewählt ist.

17. Verfahren gemäß einem der Ansprüche 9 bis 16, worin die resultierende Mischung der Bildung eines Granulats mit einer durchschnittlichen Granulatgröße von wenigstens 100 µm, bevorzugt im Bereich von 100 µm bis 2 mm unterworfen wird.

18. Verfahren gemäß einem der Ansprüche 9 bis 17, worin die kombinierte Gesamtmenge aus dem Exzipienten, Verdünnungsmittel und Träger 5 bis 99, bevorzugt 50 bis 99 Gew.-% der pharmazeutischen Zusammensetzung beträgt.

19. Verfahren gemäß einem der Ansprüche 9 bis 18, worin Desmopressinacetat verwendet und mit dem Exzipienten, Verdünnungsmittel oder Träger in einer Menge vermischt wird, die 20 bis 600 µg Desmopressinacetat pro Einheit der festen Arzneiform liefert.

## Revendications

1. Composition pharmaceutique sous une forme posologique solide comprenant de la desmopressine, ou un sel pharmaceutiquement acceptable de celle-ci, comme principe thérapeutiquement actif en même temps qu'un excipient, diluant ou support pharmaceutiquement acceptable ou un mélange de ceux-ci, **caractérisée en ce qu'**au moins l'un dudit excipient, diluant et support est un disaccharide, ledit disaccharide ayant une taille moyenne de particules dans la plage de 70 à 500 µm.

2. Composition pharmaceutique selon la revendication 1, dans laquelle ladite taille moyenne des particules est dans la plage de 75 à 350 µm.

3. Composition pharmaceutique selon la revendication 2, dans laquelle ladite taille moyenne de particules est dans la plage de 100 à 200 µm.

4. Composition pharmaceutique selon la revendication 3, dans laquelle ladite taille moyenne de particules est dans la plage de 120 à 180 µm.

5. Composition pharmaceutique selon l'une quelconque des revendications 1 à 4, dans laquelle ledit disaccharide est un lactose, de préférence le lactose-α-monohydrate.

6. Composition pharmaceutique selon l'une quelconque des revendications 1 à 5, dans la quantité totale combinée dudit excipient, diluant et support est de 5 à 99, de préférence de 50 à 99, pour cent en poids de la composition pharmaceutique.

7. Composition pharmaceutique selon l'une quelconque des revendications 1 à 6, dans laquelle ladite forme posologique solide est un comprimé destiné à être administré par voie orale qui est éventuellement adapté pour l'administration oromucosale, de préférence buccale et/ou sublinguale.

8. Composition pharmaceutique selon l'une quelconque des revendications 1 à 7, qui comprend de la desmopressine acétate en une quantité de 20 à 600 µg par unité de forme posologique solide.

9. Procédé de fabrication d'une composition pharmaceutique sous une forme posologique solide comprenant de la desmopressine, ou un sel pharmaceutiquement acceptable de celle-ci, comme principe thérapeutiquement actif, dans lequel ledit procédé comprend les étapes consistant à :
i) mélanger la desmopressine et un excipient, diluant ou support, ou un mélange de ceux-ci, éventuellement en présence d'un agent mouillant, au moins un dudit excipient, diluant et support étant un disaccharide, ledit disaccharide ayant une taille moyenne de particules dans la plage de 70 à 500 µm ;
ii) mettre le mélange résultant sous la forme de granulés, éventuellement en présence d'un agent mouillant, approprié pour la compression dans ladite forme posologique solide ;
iii) réaliser éventuellement ledit mélange et/ou la formation de granulés en présence d'au moins un additif choisi parmi un agent désintégrant, un lubrifiant, un liant, un agent odoriférant, un conservateur, un colorant et un mélange de ceux-ci ;
iv) éventuellement sécher lesdits granulés ;
v) comprimer lesdits granulés dans la forme posologique solide.

10. Procédé selon la revendication 9, dans lequel ladite taille moyenne des particules est dans la plage de 75 à 350 µm.

11. Procédé selon la revendication 10, dans lequel ladite taille moyenne des particules est dans la plage de 100 à 200 µm.

12. Procédé selon la revendication 11, dans lequel ladite taille moyenne des particules est dans la plage de 120 à 180 µm.

13. Procédé selon l'une quelconque des revendications 9 à 12, dans laquelle ledit disaccharide est un lactose, de préférence le lactose-α-monohydrate.

14. Procédé selon l'une quelconque des revendications 9 à 13, dans lequel ladite forme posologique solide est un comprimé destiné à être administré par voie orale qui est éventuellement adapté pour l'administration oromucosale, de préférence buccale et/ou sublinguale.

15. Procédé selon l'une quelconque des revendications 9 à 14, dans lequel lesdites étapes de mélange et de formation de granulés sont réalisées dans une seule macbine intégrée qui est adaptée à un tel procédé combiné.

16. Procédé selon l'une quelconque des revendications 9 à 15, dans lequel ledit agent mouillant est choisi parmi l'eau et un mélange d'eau et d'un alcool, de préférence l'éthanol.

17. Procédé selon l'une quelconque des revendications 9 à 16, dans lequel ledit mélange résultant est mis sous la forme de granulés avec une taille moyenne de particules d'au moins 100 µm, de préférence dans la plage de 100 µm à 2 mm.

18. Procédé selon l'une quelconque des revendications 9 à 17, dans lequel la quantité totale combinée dudit excipient, diluant et support est de 5 à 99, de préférence de 50 à 99, pour cent en poids de la composition pharmaceutique.

19. Procédé selon l'une quelconque des revendications 9 à 18, dans lequel on utilise la desmopressine acétate et on la mélange avec l'excipient, diluant ou support en une quantité qui fournit de 20 à 600 µg de desmopressine acétate par unité de forme posologique solide.
